# EUROPEAN PATENT SPECIFICATION

(11) **EP 1 981 518 B1**
(45) Date of publication and mention of the grant of the patent: **22.05.2013**
(21) Application number: 06779906.4
(22) Date of filing: 11.07.2006
(51) Int. Cl.: A61K 36/19, A61P 25/34, A61P 3/04

(54) **PEGANINE ISOLATED FROM ANISOTES TRISULCUS L. AS A SMOKING DETERRENT AND ANORXIGENIC AGENT**
AUS ANISOTES TRISULCUS L. ISOLIERTES PEGANIN ALS MITTEL GEGEN RAUCHEN UND ANOREXIE
PEGANINE ISOLEE A PARTIR DE ANISOTES TRISULCUS L. UTILISEE COMME AGENT DE DISSUASION TABAGIQUE ET COMME AGENT ANOREXIGENE

(43) Date of publication of application: 22.10.2008
(73) Proprietor: Al-Rehaily, Adnan Jathlan, Riyadh 11451 (SA); Al-Said, Mansour Sulaiman, Riyadh 11451 (SA); Eltahir, Kamal el Din Hussein, Omdorman (SD)
(72) Inventor: Al-Rehaily, Adnan Jathlan, Riyadh 11451 (SA); Al-Said, Mansour Sulaiman, Riyadh 11451 (SA); Eltahir, Kamal el Din Hussein, Omdorman (SD)
(74) Representative: Apostol, Salomia
(86) International application number: PCT/IB2006/002059
(87) International publication number: WO 2008/007163

(56) References cited:
- WO-A-00/48445
- GUPTA O P ET AL: "PHARMACOLOGICAL INVESTIGATIONS OF VASICINE AND VASICINONE THE ALKALOIDS OF ADHATODA VASICA" INDIAN JOURNAL OF MEDICAL RESEARCH, INDIAN COUNCIL OF MEDICAL RESEARCH, NEW DEHLI, IN, vol. 66, no. 4, 1 October 1977 (1977-10-01), pages 680-691,691A, XP000605970 ISSN: 0019-5340
- SEHGAL S K ET AL: "BROMHEXINE" INDIAN PEDIATRICS, INDIAN PEDIATRIC, CALCUTTA,, IN, vol. 27, no. 5, 1990, pages 479-483, XP008007624 ISSN: 0019-6061
- BAGCHI ET AL: "Vasaka: An important medicinal plant" JOURNAL OF MEDICINAL AND AROMATIC PLANT SCIENCES, vol. 25, no. 4, June 2004 (2004-06), XP018000590
- HERRMANN A ET AL: "Adhatoda vasica - Justicia adhatoda: Rediscovering an old herbal plant" 2006, ZEITSCHRIFT FUR PHYTOTHERAPIE 2006 GERMANY, VOL. 27, NR. 6, PAGE(S) 306-310 , XP009082769 ISSN: 0722-348X the whole document
- AL-HARBI M M ET AL: "PRELIMINARY PHYTOCHEMICAL AND PHARMACOLOGICAL STUDIES ON ANISOTES-TRISULCUS" INTERNATIONAL JOURNAL OF PHARMACOGNOSY, SWETS & ZEITLINGER, LISSE, NL, vol. 30, no. 2, 1992, pages 87-92, XP009076180 ISSN: 0925-1618
- LANHERS M C ET AL: "INFLUENCE OF ANISOTES TRISULCUS AND CREPIS RUEPPELLII EXTRACTS ON SITES OF BILE FORMATION IN THE RAT" ARZNEIMITTEL FORSCHUNG. DRUG RESEARCH, ECV EDITIO CANTOR VERLAG, AULENDORF, DE, vol. 36, no. 5, 1986, pages 826-829, XP009076201 ISSN: 0004-4172
- FLEURENTIN J ET AL: "HEPATOPROTECTIVE PROPERTIES OF CREPIS RUEPPELLII AND ANISOTES TRISULCUS: TWO TRADITIONAL MEDICINAL PLANTS OF YEMEN" JOURNAL OF ETHNOPHARMACOLOGY, ELSEVIER SCIENTIFIC PUBLISHERS LTD, IE, vol. 16, no. 1, 1986, pages 105-111, XP009076172 ISSN: 0378-8741
- AWADH A N A ET AL: "SCREENING OF YEMENI MEDICINAL PLANTS FOR ANTIBACTERIAL AND CYTOTOXIC ACTIVITIES" JOURNAL OF ETHNOPHARMACOLOGY, ELSEVIER SCIENTIFIC PUBLISHERS LTD, IE, vol. 74, no. 2, February 2001 (2001-02), pages 173-179, XP009076171 ISSN: 0378-8741
- AL-REHAILY ET AL: "Trisulcusine: A novel spiro quinazoline alkaloid from Anisotes trisulcus" MEDICINAL & AROMATIC PLANTS ABSTRACTS, NATIONAL INSTITUTE OF SCIENCE COMMUNICATION AND INFORMATION, IN, vol. 25, no. 1, February 2003 (2003-02), XP018006604 ISSN: 0250-4367

## Description

### Introduction

Throughout the world, various people almost 20% of the whole population enjoy tobacco actions via either smoking [cigarettes, pipes or shisha (nargilla)] or its sublingual intake under the tongue in a semi-dry form moistened with sodium bicarbonate (or as snuff) e.g. in Sudan and some other African countries (1).

Chronic consumption of tobacco can produce two types of adverse reactions, namely cancer of lungs and throats when it is smoked due to the tar components and a group of diseases such as hypertension, arrhythmias, hyperlipidemias, asthma and gastric and duodenal ulcers together with development of dependence with the consequence maintenance of intake due to its nicotine content (1-3). These disorders usually predispose to death.

Nicotine usually mediates its actions via two types of main receptors located at two sites in the body namely postsynaptically in the neuromuscular junction or the skeletal muscles and in the neuronal tissues peripherally and centrally at the ganglia and neurons. It excites these receptors to release acetylcholine (4). The neuronal nicotinic receptors are ion-gated channels each of which is composed of 17 subunits. Ten of them are α type, 4 are β type 2 together with γ, δ and ε (epsilon) (4). The most important receptor that is involved in nicotine dependence and the support of maintenance of nicotine intake in its various forms is the one containing the subunits α₄ β₂ (5). Activation of these receptors releases dopamine via release of acetylcholine in the nucleus accumbens and the frontal cortex (6). The release of dopamine plays an important part in nicotine-induced dependence, toxic actions and addiction (7).

Many investigators throughout the world are involved in researches aiming for discovering of drugs to help in cessation of tobacco intake in its various forms. This patent describes for the first time a chemical namely the alkaloid peganine isolated from the aerial parts of *Anisotes trisulcus* (Forssk.) Vahl family *Acanthaceae* collected from the Southern region of Saudi Arabia (8) that proved to suppress nicotine consumption and thereby opening the avenue to place itself as one of the limited armenatum available today to help in cessation of tobacco intake.

Peganine is the major quinazoline alkaloid isolated from *Anisotes trisulcus* (Forssk.) Vahl (represented 23.26% yield from AT-2 fraction). The alkaloid is also previously isolated from different plant species (9-13). Besides peganine, the plant produced other alkaloidal constituents such as anisotine, vasicinone, (14), trisulcusine and methoxypeganine (15). *Anisotes trisulcus* (Forssk.) Vahl is a shrub locally known as Almodh (16) and is a traditional herbal medicine in the Arabian Peninsula and found its way In the folk medicine of Saudi Arabia as an antidiabetic. The plant is also used as treatment for all hepatic conditions including hepatitis, jaundice and other hepatic problems (17-19). The pharmacognostical studies on the leaf of the plant have also been investigated (20). The alkaloid peganine was reported to possess abortifacient and uterine stimulant activity (21-23). In addition, it was shown to produce slight but persistent bronchodilatation, slight hypotension and to inhibit peristalsis of isolated gut (24). Other studies showed bronchoconstriction and negative inotropic effect of peganine with reduced coronary flow in the isolated heart (24).

### Materials and Methods

### Plant material

*Anisotes trisulcus* (Forssk.) Vahl was collected in March, 2005 from the way toward Fifa Mountains, Saudi Arabia.

### Extraction

The dried aerial parts (1 kg) of A. *trisulcus* were exhaustively extracted by cold percolation with 90% EtOH. The combined EtOH extract was concentrated in vacuo at 40° C to produce a solid residue (75 g). The alcoholic extract was dissolved in 225 ml of 2% tartaric acid and extracted with CHC1₃ to remove acidic and neutral components (8 g) (Fraction AT-1). The aqueous acidic solution was then alkalinized with dilute NH₄OH to pH 9 and extracted with CH₂Cl₂ to produce 0.688 g (Fraction AT-2). The aqueous layer was made more alkaline with NH₄OH to pH 12 and extracted with EtOAc to get 0.64 g (Fraction AT-3). The remaining aqueous phase was made acidic with tartaric acid to pH 4 and then lyophilized to yield 6 g (Fraction AT-4).

### Isolation and identification of peganine

The fraction AT-2 (0.688 g) was chromatographed via chromatotron 2mm plate using EtOH absol. : CHCl₃ : NH₄OH (5: 95 : 4 drops) to afford 160 mg of peganine (23.26% yield) that was crystallized using mixture a of CHCl₃ and MeOH. The alkaloid was identified as peganine by comparing its physical and spectral data with those reported previously in our laboratory and with the literature (14).

### Effect of peganine on nicotine and food consumption In rats:

Male Wistar rats (200 g body weight) were divided at random into various groups (N = 6 animals per group). Each animal was placed in a stainless steel cage and provided with a known weight of normal rat chow food pellets and drinking battle containing a known volume of aqueous 0.25% sodium carboxymethylcellulose containing either nicotine acid tartarate to provide a final concentration of 40 µg/ml (in case of control groups) or the same concentration of nicotine plus the indicated concentration of peganine. Peganine was added in form of its emulsion in 0.25% aqueous sodium carboxymethylcellulose in volumes that provide a final concentration of 0.25% of sodium carboxymethylcellulose in the drinking fluid. The animals were allowed both food and the drinking liquid *ad libitum.* The room temperature where the animals were placed was maintained at 23 ± 2 °C. The light cycle was kept at 12 h light and 12 h dark. The relative humidity was maintained at 75 ± 5 %. Both food and drinking liquid consumptions were measured every 2 days and at the end of the treatment which continued for complete seven consecutive days. The influence of the treatment on food consumption, the drinking of fluid intake and hence nicotine consumption was calculated at the end of the treatment.

### Statistical analysis

All results were reported as their means ± s. e. mean with N = number of experiments. Statistical significance between control and treated groups were performed using Student's *t*-test.

### Results

### Effect of peganine on nicotine and food consumption in rats:

As peganine was found to be the major constituent from fraction AT-2 in this study, it was tested in rats. Treatment of the animals with peganine 1.4 mg/ml in the drinking vehicle mixed with nicotine (40 µg/ml) produced significant reductions in both nicotine and food consumptions. It decreased the nicotine consumption and food intake by 79 and 77.8%, respectively. The details are shown in table 1.

### Discussion

The results of this study revealed that peganine (vasicine or linarine) which is known chemically as 1,2,3,9 tetrahydropyrolo[2,1b]quinazolin-3-ol produced potent significant inhibitions of nicotine intake and food consumption. It exerted a potent anorexigenic action. In fact this constituent acted as a nicotine substitute.

Peganine actions are probably mediated via activation of α₄ β₂ nicotinic receptors as does nicotine leading to release of dopamine in the mesolimbic sites of the brain. Thus, it probably acted as a nicotine substitute leading to satisfaction of the rewarding system leading to an explanation of the reduced nicotine intake (25).

Generally, speaking an agonist at a certain receptor may either be a potent, low potent partial or potent partial agonist. Previous pharmacological studies revealed that, potent nicotinic agonists or low potency partial agonists would not be expected to antagonize the effects of nicotine but agents that act as potent partial agonists can antagonize nicotine (26-27). For instance varenicline, the potent partial nicotinic α₄ β₂ agonist, in a dose of 5.6 mg/kg significantly blocked nicotine (1 mg/kg)-induced increase in dopamine turnover in the mesolimbic system of rats (27). The peganine prevention of nicotine consumption and food intake suggests that peganine has probably acted as substitute for nicotine via acting through α₄ β₂ nicotine receptor.

Thus, it is highly likely that peganine can act to limit craving and withdrawal from nicotine in chronic tobacco consumers (smokers or snuff intakers). Thus, we claim that peganine has the potential of a novel treatment for tobacco dependence. In this aspect, it is hoped to be more fruitful than the partial nicotine agonist (-)-cytisine (28-29) that has poor blood brain barrier penetrability (30). It is hoped to be a new addition to the armenatum of tobacco cessation campains that include the skin patch containing both nicotine and its antagonist mecamylamine (31). The soonly expected varenicline, the partial α₄ β₂ nicotine agonist (27), the antidepressant bupropion (or amfebutamine) (32) and the nicotine conjugate vaccine (Nicvax®) (33).

Considering food intake and appetite, we can recall that the two most important brain neurotransmitters that suppress food intake are dopamine and serotonin (34). The significant inhibitions induced by peganine in food intake incline us to believe that the anorexigenic effects were mediated by dopamine release via initial activation of α₄ β₃ nicotinic receptors. Indeed activation of these receptors releases dopamine (6, 7, 25).

In conclusion, this patent showed that peganine isolated from *Anisotes trisulcus* (Forssk.) Vahl family *Acanthaceae* possessed properties that enabled it to suppress nicotine consumption and food intake. Thus, we claim:
1- The use of peganine for the manufacture of a pharmaceutical agent for suppression of nicotine consumption.
The non-therapeutic use of peganine for reduction of food intake.

### Acknowledgement

We would like to thank Professor Farouk S. El-Feraly for his kind suggestions and encouragements. We would also like to thank the College of Pharmacy, King Saud University for providing the facilities to carry out this work.

### References

1- ELTahir, Kamal E.H. (1985). Cigarettes, Snuff and Health (1984) Publisher: Sudan Council of Research, Khartoum, Sudan.
2- Taylor, A.L. and Bettcher, D.W. (2000). Bull. World Health Organization 78, 920-929.
3- Doll, R., Peto, R., Boreham, J. and Sutherland, I. (2004). Mortality in relation to smoking: 50 years observations on male British Doctors. Br. Med. J. 328, 1519-1527.
4- Sargent, P.B. (2000). The distribution of neuronal nicotinic ACh receptors (eds. Clementi, F., Fomassari, D. and Gotti, C.) Publisher: Springer-Verlag, Berlin, Heidelberg, Germany, Vol. 144, 163-192.
5- Cohen, C.B., Bergis, O.E., Galli, F., Lockhead, A., Jegham, S., Biton, B., Leonardon, J., Avent, P., Sagard, F., Besnard, F., Graham, D., Coste, A., Oblin, A., Curet, O., Voltz, C, Gardes, A., Caille, D., Perrault, G., George, P., Sourbrie, P. and Scatton, B. (2003). SSR 591813, a novel selective and partial α4 β2 nicotinic receptor agonist with potential to smoking cessation. J. Pharmacol. Expt. Ther. 306, 407-420.
6- Dani, J.A. and De Biasi, M. (2001). Cellular mechanism of nicotine addiction. Pharmacol. Biochem. Behav. 70, 439-446.
7- Di Chiara, G. (2000). Role of dopamine in the behavioral actions of nicotine related to addiction. Eur. J. Pharmacol. 393, 295-314.
8- Collenette, S. (1999). Wildflowers of Saudi Arabia. Publisher: National Commission for Wildlife Conservation and Development, Riyadh, Kingdom of Saudi Arabia, p. 2.
9- Groger, D. and Johne, S. (1965). Occurrence of peganine in Linaria species. Planta Med. 13, 182.
10- Huq, M.E. Ikram, M. and Warsi, S.A. (1967). Chemical composition of Adhatoda vasica Linn II, Pak. J. Sci. Ind. Res. 10, 224-225.
11-Ghosal, S. Chauhan, R. and Mehta, R. (1975). Chemical constituents of Malvaceae. Part 1. alkaloids of Sida cordifolia. Phytochemistry, 14,830-832.
12- Kurbanov, D. and Zharekeev, B.K. (1974). Alkaloids of Beibersteinia multifida and Peganum harmala from Karakalpak assr. Chem. Comp. 10, 715.
13- Schipper, A. and Volk, O.H. (1960). The alkaloids of Peganum harmala. Dtsch. Apoth. Ztg. 100, 255.
14- Al-Azizi, M.M. (1997). Quinazoline alkaloids from Anisotes trisulcus L. Egypt J. Biotechnol. 2, 1-6.
15- Al-Rehaily, A.J. El-Sayed, K.A. Al-Said, M.S. and Ahmed, B. (2002). Trisulcusine: A novel spiro quinazoline alkaloid from Anisotes trisulcus. Indian Journal of Chemistry, 41 B, 2385-2389.
16- Al-Hubaishi, A. and Muller-Hohenstein, K. (1984). Introduction to Vegetation of Yemen, Ecological Basis, Floristic Composition, Human Influence, p.134 & 189.
17- Fleurentin, J., Hoefler, C, Lexa, A., Mortier, F. and Pelt, J.M. (1986). Hepatoprotective properties of Crepis rueppellii and Anisotes trisulcus: two traditional medicinal plants of Yemen. J. Ethnopharmacol. 16, 105-111.
18- Lanhers, M.C., Bertrand, I., Fleurentin, J., Lehr, P.R. and Pelt, J.M. (1986). J. Drug Research, 36, 826-829.
19- Al-Harbi, M.M., Al-Said, M.S. and Al-Azizi, M.M. (1992). Preliminary phytochemical and pharmacological studies on Anisotes trisulcus. Int. J. Pharmacognosy, 30, 87-92.
20- Al-Rehaily, A.J. (2000). Pharmacognostic studies on the leaf of Anisotes trisulcus (Forssk.). Pak. J. Bio. Sci. 3, 1427-1430.
21- Gupta, O.P., Sharma, M.L., Ray Ghaatak, BJ. and Atel, C.K. (1977). Potent uterine activity of alkaloid vasicine. Indian J. Med. Res. 66, 865-871.
22- Chandhoke, N., Gupta, O.P. and Atel, C.K (1978). Abortifacient activity of the alkaloid vasicine through the release of prostaglandin. J. Steroid Bioche. 9, 885.
23- Gupta, O.P., Wakhloo, R.L., Sharma, M.L. and Atel, C.K. (1979). Vasicine- apotent uterine stimulant studies on human myometrium. J. Gynaecol. India. 29, 935-938.
24- Lahiri, P.K. and Pradiian, S.N. (1964). Pharmacological investigation of vasicinol an alkaloid from Adhatoda vasica Nees, Indian J. Exp. Biol. 2, 219-223.
25- Tapper, A.R., mCkINNEY, s.l., Nashmi, R., Schwarz, J. Deshpande, P., Labarca, C., Whiteaker, P., Marks, M.K., Collins, P.C. and Lester, H.A. (2004). Nicotine actication of α4 receptors sufficient for reward, tolerance and sensitization. Science, 5, 1029-1032.
26- Stuhmer, W. (1992). Electrophysiological recording from xenopus oocysts. Methods Enzymol. 207, 319-339.
27- Coe, J.W., Brooks, P.R., Vetelino, M.G., Wirtz, M.C., Arnold, E.P., Huang, L, Sands, S.B., Davis, T.I., Lebel, L.A., Fox, C.B., Shrikhande, A., Heym, J.H., Schaeffer, E., Rollema, H., Lu, Y., Mansbach, R.S., Chambers, L.K., Rovetti, C.C., Schultz, D.W., Tingley, D., and O'Neil, B.T. (2005). Varenicline: an α4 β2 nicotinic receptor partial agonist for smoking cessation, J. Med. Chem. 48, 3474-3477.
28- Barlow, R.B. and McLeod, L.J. (1969). Some studies on cytisine and its methylated derivative, Br. J. Pharmacol. 35, 162-174.
29- Scharfenberg, G., Benndort, S. and Kempe, G. (1971). Cytisine [TABEX]R als. Medikamentose Rancherent wo hungshilfe. Dtsch Gesund heitsw 26, 463-465.
30- Reavill, G., Walther, B., Stolerman, LP. and Testa, B. (1990). Behavioural and pharmacokinetics studies on nicotine cytosine and lobeline. Neuropharmacology 29, 619-624.
31- Rose, J.E., Behm, F.M. and Westman, E.C. (1994). Mecamylamine combined with nicotine skin patch facilitates smoking cessation beyond nicotine patch treatment alone. Clin. Pharmacol. Ther. 56, 86-99.
32- Warner, C-, and Shoaib, M. (2005). How does bupropion work as a smoking cessation aid? Addiction Biology 10, 219-223.
33- Fattom, A., Ennifar, S., Horwith, G., Fuller, S, Pental, P., Malin, D. and Naso, R. A nicotine conjugate vaccine for the possible treatment and prevention of nicotine addiction. 225th ACS National Meeting, New Orleans, LA, USA. March, Abstract Book 23-27.
34- Silverstone, T. (1992). Appetite suppressants. Drugs 43-820-36.

**Table 1. Effect of peganine (vasicine) on nicotine and food consumption in rats**

| Treatment | Concentration of nicotine in drinking water µg/ml | Liquid consumed daily per 200 g rat per day ml | Quantity of nicotine consumed per day mg | % decease | Quantity of food consumed per 200 g rat per day g | % decease |
|---|---|---|---|---|---|---|
| Control | 40 | 26.25 ± 0.9 | 1.05 | - | 25.5 ± 1.4 | - |
| Peganine | 40 | 5.5 ± 0.13* | 0.22 | 79 | 5.66 ± 0.3* | 77.8 |

| | | | | | | |
|---|---|---|---|---|---|---|
| P* < 0.01, N = 6 | | | | | | |

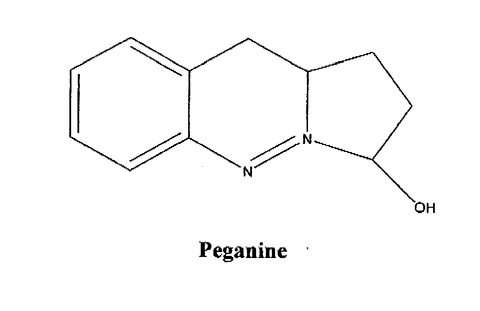

## Claims

1. The use of peganine for the manufacture of a pharmaceutical agent for suppersion of nicotine consumption.

2. The non-therapeutic use of peganine for reduction of food intake.

## Patentansprüche

1. Die Verwendung des Peganines für die Herstellung eines pharmazeutiches Agens für der Nikotinverbrauch beseitigen.

2. Die nicht therapeutische Verwendung des Peganines fur der Nahrungsaufnahme Reduzierung.

## Revendications

1. L'utilisation de peganine pour la fabrication d'un agent pharmaceutique pour la consommation de nicotine suppersion

2. L'utilisation non thérapeutique de peganine pour la réduction de la prise alimentaire
